(19) Europäisches Patentamt
European Patent Office
Office européen des brevets

(11) Publication number : **0 098 707 B1**

# (12) EUROPEAN PATENT SPECIFICATION

(45) Date of publication of patent specification : **02.01.86**

(21) Application number : **83303539.7**

(22) Date of filing : **20.06.83**

(51) Int. Cl.[4] : **C 07 B 57/00**, B 01 D 15/08, C 08 G 77/02// C07C59/50, C01G51/00

(54) **Material for resolution of optical isomers, its preparation and use.**

(30) Priority : **30.06.82 JP 113346/82**

(43) Date of publication of application : **18.01.84 Bulletin 84/03**

(45) Publication of the grant of the patent : **02.01.86 Bulletin 86/01**

(84) Designated contracting states : **DE FR GB SE**

(56) References cited :
**CH-A- 509 239**
**JOURNAL OF CHROMATOGRAPHY, vol. 185, 20th December 1979, pages 361-373, Elsevier Scientific Publishing Comp., Amsterdam. NL., f.K. CHOW et al.: "High-performance liquid chromatography of nucleotides and nucleosides using outersphere and innersphere metal-solute complexes"**
**CHEMICAL ABSTRACTS, vol. 84, no. 23, 7th June 1976, page 405, no. 164073m, Columbus, Ohio, USA, J. GAAL et al.: "Chromatographic separation of optical isomers by means of outer-sphere complex formation reactions"**

(73) Proprietor : **Seiwa Technological Laboratories, Limited**
**4-19-10 Azusawa Itabashi-ku**
**Tokyo (JP)**

(72) Inventor : **Muto, Masayuki**
**1053-9, Nakasho**
**Izumisano-shi Osaka-fu (JP)**

(74) Representative : **Gibson, Christian John Robert et al**
**Marks & Clerk 57/60 Lincoln's Inn Fields**
**London WC2A 3LS (GB)**

EP 0 098 707 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue St-Denis, 75001 Paris, France

**Description**

## Background of the Invention

This invention relates to a novel material capable of optically resolving with good efficiency a mixture of optically active compounds, and which for example can be used to resolve a racemic mixture.

## Prior Art

In the prior art, a main chromatographic procedure for optical resolution of racemic mixtures involves ligand exchange chromatography. This method comprises packing a column with a filler having a chiral amino acid bonded to a synthetic resin. The filled column is then saturated with metallic ions and a mixture of optically active compounds to be resolved is passed as a solution through the column. Separation occurs into the respective optically active compounds. This method is based in principle on the difference in coordinating forces of the optically active isomers to the metallic ions in the resin. With this prior art method, however, there are drawbacks, in that the applicable samples are restricted to compounds having groups capable of acting as ligands, such as amines, amino acids or carboxylic acids. Moreover, the complexes formed in the synthetic resin layer are labile and elimination reactions may occur. It is also to be noted that only water soluble samples may be used and that in practice both samples and eluents have to be aqueous electrolyte solutions.

Another resolution procedure uses an inert optically active metal complex ion adsorbed onto a cation exchange resin. However, in this method, the bonding between the metal complex ion and the ion exchange resin is a mere physical adsorption. Therefore, elimination of the complex ion from the resin can readily occur with various eluents. Accordingly, there are restrictions on the possible compounds which can be resolved, and again the method is applicable only to water soluble samples : both the samples and the eluents have to be aqueous electrolyte solutions.

## Objects of the Present Invention

One object of this invention is to provide a material for resolution of optical isomers. Such a material should be applicable for samples of electrolytes as well as for samples of non-electrolytes. Thus, a further object of this invention is to provide a resolution material which can be used in either an aqueous solvent or a non-aqueous solvent such as an organic solvent.

A related object of this invention is to devise a synthesis for new resolution materials, while an additional object is to put forward a method of resolving optical isomers which method can be used efficiently with many kinds of optically active compound.

## Summary of the Invention

It has been found that an optically active complex covalently bonded to silicon dioxide can afford a material suited for resolution of optical isomers. More specifically, the material comprises macromolecular silicon dioxide, for example silica gel, to which are chemically bonded molecules of a resolved optically active form of a cobalt(III) complex. Such a material can be prepared by modification of available synthetic methods, and can give good resolution of optically active compounds, especially organic compounds.

## Description of Preferred Embodiments

The cobalt(III) complex molecules are covalently bonded to the macromolecular silicon dioxide, and are thus chemically secured in position. Such bonding can be achieved through bridging chains. Each bridging chain can extend to the macromolecular silicon dioxide from a ligand on the complex, and typically includes one or more carbon atoms and a silicon atom. The silicon atom of the bridging chain can then link the carbon atoms of the chain to the macromolecular silicon dioxide, usually through a silanol bond. The complex suitably has six amino nitrogen atoms coordinated thereto in order to impart optical activity, and it is a simple matter to arrange that the bridging chain extends from one of the six coordinating amino nitrogen atoms.

Thus, in one aspect of this invention there is provided a material for optical resolution of a mixture of optical isomers of an optically active compound which material comprises chromatographic silicon dioxide and a resolved optically active cobalt(III) complex. The complex is an optically active hexamine type complex with six amino nitrogen atoms coordinated to a cobalt(III) centre. A carbon chain is attached to one of the six nitrogen atoms, and in this aspect of the invention the carbon chain connects the cobalt(III) complex to the silicon dioxide through a silanol-type bond.

For preference, the cobalt(III) complex of the resolution materials of this invention comprises an optically active cation which can be represented by the formula (1) :

$$[Co(biN_2)(coordN_4)]^{3+} \qquad (1)$$

wherein $biN_2$ represents a bidentate ligand, the bidentate ligand having two amino nitrogen atoms coordinated to the cobalt(III) atom and the bidentate ligand carrying on one of its nitrogen atoms the bridging chain (not shown in the formula) to the macromolecular silicon dioxide ; and $coordN_4$ represents four amino nitrogen atoms coordinated to the cobalt(III) atom and forming part of two bidentate ligands or one tetradentate ligand.

The molecular formula of the resolution materials of this invention is not certain, but they can be considered to be of the general formula (2) :

$$(Si\text{-}O\text{-}Si\text{-})\text{-}A\text{-}[Co(biN_2)(coordN_4)]^{3+} \cdot X \qquad (2)$$

wherein :

(Si-O-Si-) represents a silicon dioxide macromolecule bonded through a silicon atom to the group A ;

A represents a divalent hydrocarbyl group, preferably a saturated aliphatic hydrocarbyl group ;

$Co(biN_2)(coordN_4)$ is a resolved optically active cobalt(III) complex in which $biN_2$ represents a bidentate ligand, the bidentate ligand having two amino nitrogen atoms coordinated to the cobalt(III) atom and the bidentate ligand carrying on one of its nitrogen atoms the group A and $coordN_4$ represents four amino nitrogen atoms coordinated to the cobalt(III) atom and forming part of two bidentate ligands or one tetradentate ligand ; and

X represents one or more anions satisfying the cationic charge of 3 +.

Any uncertainty in structure arises mainly from the nature of the macromolecular portion and the way it bonds to the bridging group A. Moreover the given formula does not explicitly show more than one cobalt(III) complex linked to the macromolecule. Nevertheless, the general formula (2) is of assistance in discussing the invention and will be used in the non-limiting sense.

A preferred group of compounds of this invention may then be exemplified by compounds having the general formula (2a) :

$$(Si\text{-}O\text{-}Si\text{-})\text{-}A\text{-}\Delta\text{-}[Co(biN_2)(coordN_4)]^{3+} \cdot X \qquad (2a)$$

or by the formula (2b) :

$$(Si\text{-}O\text{-}Si\text{-})\text{-}A\text{-}\Lambda\text{-}[Co(biN_2)(coordN_4)]^{3+} \cdot X \qquad (2b)$$

wherein (Si-O-Si-), A, $biN_2$, $coordN_4$ and X are as defined above, and the greek symbols are used in the conventional manner to indicate the stereochemistry, as described for example in Inorganic Chemistry *9* (1970) at page 1.

Among the compounds of formula (2) according to this invention, a typical example has the following formula :

$$(Si\text{-}O\text{-}Si\text{-})\text{-}(CH_2)_3\text{-}\Delta\text{-}[Co(en)(phen)_2] \cdot Cl_3,$$

which exists in two forms

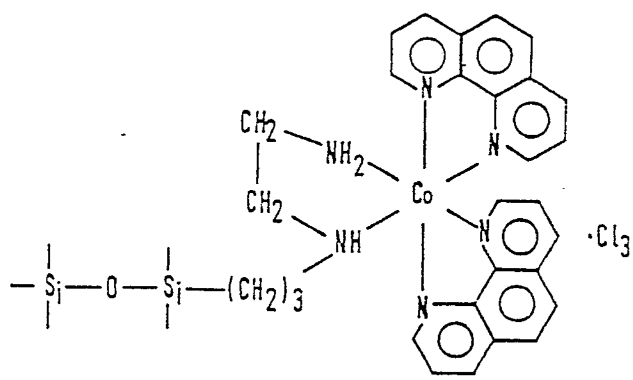

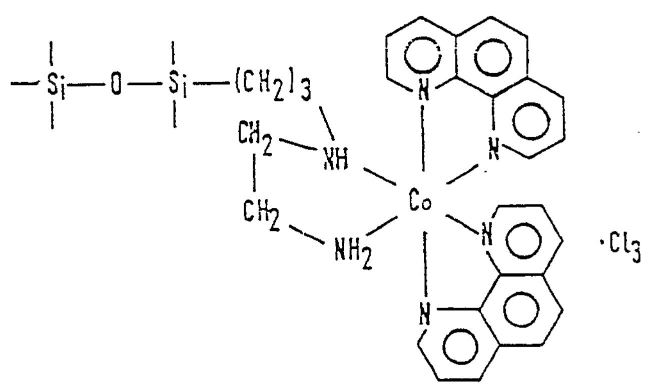

In the compounds of this invention there is a carbon chain included in the covalent bridge between the resolved cobalt complex and the silicon-containing macromolecule. This carbon chain is usually a divalent hydrocarbyl group with a backbone of 2 to 4 carbon atoms, and can have a saturated or unsaturated acyclic, cyclic or mixed acyclic/cyclic chain. Suitable hydrocarbyl groups include, for example, alkylene groups, alkenylene groups, phenylene, 4,4'-biphenylene or other arylene groups, and cyclohexylene or other cycloalkylene groups. Particularly preferred are straight chain lower alkylene groups of 2 to 4 carbon atoms, such as ethylene or trimethylene.

The bidentate ligand bi $N_2$ in the preferred compounds of this invention preferably has 2 or 3 carbon atoms between the two amino nitrogen atoms, and is preferably an alkylenediamine, cycloalkylenediamine or arylenediamine group, for example an ethylenediamine, trimethylenediamine, 1,2-propanediamine, 1,3-propanediamine, cyclohexanediamine, or phenylenediamine, though its nature is not critical. Ethylenediamine is particularly preferred.

The substituent coord$N_4$ can be two bidentate ligands, such as the bidentate ligands given above for the ligand bi$N_2$ and again preferably has 2 or 3 carbon atoms between the nitrogen atoms. The diamine bidentate ligands such as ethylenediamine, d- and/or 1-1,2-propanediamine are preferred, but there may also be mentioned piperidine, phenanthroline or other nitrogen heterocyclic compounds, and trans-cyclohexanediamine as preferred examples. The substituent coord$N_4$ can also be a tetradentate ligand, preferably with 2 or 3 carbon atoms between each nitrogen atom, and is then, for example, triethylenetetramine.

The one or more anions X satisfying the cationic charge of 3+ may include those anions employed conventionally in complex compounds : preferred anions include a halogen ion such as bromide ion or chloride ion, a sulphate ion, and a chlorate ion.

The resolution materials of this invention can be prepared by resolving an optically active cobalt(III) complex (3) having four coordinated amino nitrogen atoms and two coordinated labile atoms to give a resolved isomer ; the four amino nitrogen atoms forming part of two bidentate ligands or one tetradentate ligand, and the two labile atoms forming part of two monodentate labile ligands or one bidentate labile ligand, each labile ligand being capable of ligand exchange with a bifunctional group having two coordinatable nitrogen atoms ; and reacting the resolved isomer of the optically active cobalt(III) complex in a solvent with a macromolecular silicon dioxide functionalized with an organic group having two nitrogen atoms capable of forming a bidentate ligand on the cobalt(III) complex.

Preferred cobalt complexes of formula (3) for use as starting compounds in the reaction may be represented by the formula (4) :

$$[CoL_2coordN_4]Y \qquad (4)$$

wherein $L_2$ represents the ligands coordinated through labile atoms, coord$N_4$ is as defined above, and Y represents one or more anions satisfying the cationic charge of the complex.

Examples of appropriate ligands L include diaqua, diacido, dinitrato, carbonato, and other replaceable ligands as are well known in the art. Y will usually be a sulphate ion, chlorate ion or a halogen ion such as a chloride or bromide ion.

Thus, examples of preferred starting materials of formula (3) include the following compounds :

$$[Co(en)(chda)_2]\ X_3$$

$$[Co(en)(pn)_2]\ X_3$$

$$[Co(en)(bip)_2]\ X_3$$

$$\alpha(\text{or } \beta)[Co(en)(trien)]\ X_3$$

wherein chda represents trans-cyclohexanediamine, pn represents d (or 1)-1,2-propanediamine, bip represents bipyridine and trien represents triethylenetetramine.

Resolution of the complex (3) can be achieved using conventional procedures reported in the literature and for instance involving diastereoisomer formation. The functionalized macromolecular silicon dioxide is preferably one represented by the notional formula (5) :

$$(Si\text{-}O\text{-}Si\text{-})\text{-}A\text{-}biN_2 \qquad (5)$$

wherein (Si-O-Si-), A and bi$N_2$ are as defined above. Such a starting material can easily be prepared using available synthetic procedures, such as those described in J. Chromatogr. *185* (1979) 361 for the compound referred to as « Si-en ». Thus, macromolecular silicon dioxide can be reacted with a siloxane compound which is preferably of the formula (7) :

$$(Ro)_3\text{-}Si\text{-}A\text{-}NH\text{-}A'\text{-}NH_2 \qquad (7)$$

wherein R is an alkyl group, usually a methyl or ethyl group, A is as defined above and A' is a divalent

hydrocarbyl group, usually with a backbone of 2 or 3 carbon atoms between the nitrogen atoms. The part-structure $-NH-A'-NH_2$ corresponds with the group $biN_2$ defined above which is preferably alkylenediamine, cycloalkylenediamine or arylenediamine group, especially an ethylenediamine, trimethylenediamine, 1,2-propanediamine, 1,3-propanediamine, cyclohexanediamine or phenylenediamine.

Examples of suitable solvents for the reaction between the compound (3) and the compound (5) include polar non-proton solvents, particularly the substituted acid amine solvents such as dimethylformamide. The reaction temperature and the time required for the reaction may vary depending primarily on the particular nature of the starting compounds employed. It is preferred to carry out the reaction at a relatively low temperature, for example, at room temperature or below, even down to the freezing point of the solvent or lower still, in order to suppress the racemization reaction. The time required for the reaction is normally one to several or many days. In this reaction it seems the silicon atom in the compound (7) becomes a silanol group bonding the group A to the macromolecule, thus giving the partstructure M-O-Si-A- (where M is the macromolecule and A is as defined). After completion of the reaction, the desired compound may be isolated using conventional procedure such as filtration under reduced pressure and purified similarly.

The resolution materials of this invention can be used for optical resolution of a mixture of optical isomers of an optically active compound by contacting the mixture with the resolution material and sequentially separating the isomers from the material.

Optical resolution with the present resolution materials can be carried out by using conventional procedures such as column chromatography. A convenient resolution technique involves packing a column with the material of this invention, charging the column with a solution of the substance to be resolved, and eluting with a suitable eluent. In particular, high speed liquid chromatography equipment can readily be employed for the resolution method of this invention.

The use of a material similar to those of the present invention in a chromatographic system is described in J. Chromatogr. *185* (1979) 361. However, in this previous article the material is employed for separating nucleotides and nucleosides from each other. To this end, the cobalt complex was employed in its racemic form, with no suggestion of using an optically resolved form. The known material is employed chromatographically to separate different compounds from each other.

The method according to this invention is one of optical resolution and is widely applicable to various natural products, synthetic compounds, and other substances. For example, it can be effectively applied to organic compounds such as amino acids, carboxylic acids, nucleic acids, steroids, and perfumes.

Furthermore, the typical optical resolution material according to this invention is relatively inert. Since part of one of the ligands on the optically active cobalt centre is chemically bonded to the silicon dioxide, little if any racemization or decomposition occurs in the complex itself. Additionally, because the resolution involves hydrogen bonding or pi-pi interaction between the complex and the compound being resolved, few restraints are placed on the compounds which can be resolved. The resolution material of this invention can thus be used on a wide range of samples substantially without restriction on the aqueous or non-aqueous properties of the permitted eluents or the hydrophilic or hydrophobic properties of the resolvable compounds.

## Description of the Drawings

Figure 1 is the circular dichroism (« CD ») spectrum of the resolution material prepared in Example 1, a compound termed
« (Si-O-Si-)-(CH)-Δ)[Co(en)-(phen)$_2$]$Cl_3$ » ; and

Figure 2 is a high speed liquid chromatogram for the optical resolution of DL-mandelic acid using the resolution material of Example 2

## Examples of the Invention

This invention is further illustrated in the following non-limiting Examples.

### Example 1

« (Si-O-Si-)-$(CH_2)_3$-Δ-[Co(en)(phen)$_2$]$Cl_3$ »

a) [$CoCO_3$(phen)$_2$]Cl, $4.5H_2O$

This compound was prepared by a literature method [Bull. Cher. Soc. Jpn. *49* (1976) 1573]

Thus, 12 ml of 30 v/v % aqueous hydrogen peroxide was added to an ice-cooled and stirred solution of 14.2 g cobalt chloride $CoCl_2 \cdot 6H_2O$ in 30 ml water. An ice-cooled solution of 36 g potassium hydrogen carbonate in 36 ml water was then added and the resultant mixture stirred for 30 minutes. 30 ml of 50 v/v % aqueous ethanol and 21.6 g 1,10-phenanthroline were added, and the resulting mixture stirred for one

hour at 20 °C and then for two hours at 30 to 35 °C. 30 ml water was added, the temperature raised to 50 °C, and stirring was continued for one hour. Subsequently, a mixture of 7.2 g glacial acetic acid and 30 ml water was added dropwise over at least 15 minutes and followed by stirring for 2 hours. This dropwise addition of diluted glacial acetic acid was repeated. After further stirring of the mixture for 30 minutes, a precipitate was filtered off. Further precipitate was obtained after the filtrate had been left to stand overnight in a refrigerator and was filtered off. The resultant red solid was washed with aqueous acetone, then with acetone, and dried in air to obtain 25.9 g of the desired compound $[CoCO_3(phen)_2]Cl$, $4.5H_2O$ in 86 % yield.

b) Δ-$[CoCO_3(phen)_2]Cl$, $4.5H_2O$

5 g of the $[CoCO_3(phen)_2]Cl$, $4.5H_2O$ obtained in step (a) above was dissolved in 20 ml water at 50 °C and a solution of 2.0 g of potassium antimonyl d-tartarate $K_2[Sb_2(C_4H_2O_6)_2] \cdot 3H_2O$ in 15 ml water at 65 °C was added thereto. After homogeneous stirring, the mixture was left to stand in a refrigerator overnight. The insolubles were filtered off and the filtrate freeze-dried. Methanol was added to the dried material, insolubles filtered off, and ether added to the filtrate. The resultant mixture was filtered and the precipitate washed with ether and dried in air. In this way the resolved isomer Δ-$[CoCO_3(phen)_2]Cl \cdot 4.5H_2O$ was obtained.

c) « (Si-O-Si)-$(CH_2)_3$-NH-$(CH_2)_2$-$NH_2$ »

This compound in which an ethylenediamine function is linked to macromolecular silicon dioxide was prepared in accordance with the published method of J. Chromatogr. *185* (1979) 361. The compound is also referred to herein as « Si-en ».

5.0 g of a silica gel with pore diameter 85 Angstroms (Partisil®-10 produced by Whatman Co) was vacuum dried at 150 °C for 2 hours and added to a solution of 35 ml of 3-(2-aminoethylamino)propyltrimethoxysilane $(CH_3O)_3Si$-$(CH_2)_3$-NH-$(CH_2)_2$-$NH_2$ in 65 ml dry toluene. The resultant mixture was heated at reflux for 6 days while passing nitrogen gas into the mixture. After cooling, the mixture was filtered and the precipitates collected, washed in sequence with toluene, isopropanol, acetone, methanol and again acetone, and then the washed material was vacuum dried at 100 °C for 2 hours to give a pale yellow material, identified as « Si-en ».

d) « (Si-O-Si)-$(CH_2)_3$-Δ-$[Co(en)(phen)_2]Cl_3$ »

7 g of the Δ-$(CoCO_3(phen)_2)Cl_3$ obtained in step (b) above was added to 100 ml of dehydrated dimethylformamide. 5.5 g of the « Si-en » obtained in step (c) above was added. The resultant mixture was stirred at 10 °C for 30 hours and then filtered to give a precipitate which was washed successively with dimethylformamide, water, methanol and then ether. The washed material was dried in air to obtain the desired product as a yellow-tinted scarlet material.

The CD-spectrum of this material, « (Si-O-Si-)-$(CH_2)_3$-Δ-$[Co(en)(phen)_2]Cl_3$ », was a shown in Fig 1 : the spectrum shows that the product is optically active, though a definitive interpretation of the curve is difficult in view of the unusual structure of the macromolecule.

Example 2

« (Si-O-Si-)-$(CH_2)_3$-Λ-$[Co(en)_3]Cl_3$ »

a) cis-$[CoCl_2(en)_2]Cl$

This compound was prepared using a published method (« New Course of Experimental Chemistry », volume 8 (1977), Maruzen)

Thus, to a stirred solution of 16 g $CoCl_2$ $6H_2O$ in 50 ml water was added 60 g of 10 v/v % aqueous ethylenediamine solution (60 g). The mixture was oxidized by passage of air for 15 hours, and 35 ml concentrated hydrochloric acid was added. The resultant mixture was concentrated by heating on a boiling water bath until a thin film formed on the liquid surface and was then left to cool overnight. The resultant green crystals of trans-$[CoCl_2(en)_2]Cl \cdot H_2O \cdot HCl$ were filtered off, washed successively with ethanol and ether, and then dried at 110 °C for 3 hours. Water was added to this trans-product, the mixture was concentrated by heating on a water bath, and then it was left to cool, whereby cis-$[CoCl_2(en)_2]Cl$ precipitated as violet crystals. These crystals were collected by filtration, and the filtrate further concentrated by heating to give an additional amount of the desired cis-isomer.

b) Λ-cis-$[CoCl_2(en)_2]Cl$

13 g of the fine powdered cis-$[CoCl_2(en)_2]Cl$ obtained in step (a) was quickly dissolved in 365 ml water at 17 °C and insolubles removed by filtration. The filtrate was cooled to 10 °C, 9,5 g ammonium d-α-

bromocamphorsulphonate was added, and the mixture stirred at 10 °C for 30 minutes. The resulting precipitate was collected by filtration and washed successively with ethanol and ether. This product was triturated rapidly in an ice-cooled mortar with 34 ml of an ice-cooled mixture of equal volumes of concentrated hydrochloric acid, ethanol and ether. The mixture was then filtered, and the precipitate collected, washed successively with ethanol and ether and dried in air to give powdery Λ-cis-$[CoCl_2(en)_2]Cl$.

c) Λ-cis-$[Co(NO_3)_2(en)_2]NO_3$

To 5 g of the Λ-cis-$[CoCl_2(en)_2]Cl$ obtained in the above step (b) was added 25 ml water at 5 °C, and to the resultant mixture was added a mixture of 9 g of finely ground silver nitrate in 10 ml water at 5 °C : the resultant mixture was stirred at 0 °C for 10 minutes. 10 ml of ethanol was added, the mixture filtered and the filtrate freeze dried to obtain the desired compound, scarlet Λ-cis-$[Co(NO_3)_2(en)_2]NO_3$.

d) « (Si-O-Si-)-$(CH_2)_3$-Λ-$[Co(en)_3]Cl_3$ »

80 ml dimethylformamide was added to 5,2 g of the « Si-en » obtained in Example 1 (c) above and the mixture cooled to − 20 °C. 4,8 g of the Λ-cis-$[Co(NO_3)_2(en)_2]NO_3$ obtained at step (C) of this Example 2 was added, and the mixture stirred for 5 days. The mixture was then filtered and the precipitate collected, successively washed with 6N-hydrochloric acid, ethanol and ether, and dried in air to obtain the desired compound,

« (Si-O-Si-)-$(CH_2)_3$-Λ-$[Co(en)_3]Cl_3$ », as a pale yellow material.

Example 3

Optical resolution of DL-mandelic acid

Optical resolution of racemic DL-mandelic acid was performed by use of high speed liquid chromatography (hplc) equipment (TRIROTAR-II produced by Nippon Bunko Kabushiki Kaisha) in which the hplc column was filled with the product of Example 2.

The operating details for the resolution were as follows
Sample : DL-mandelic acid 10 mg/ml in water
Filler : « (Si-O-Si)-$(CH_2)_3$-Λ-$[Co(en)_3]Cl_3$ »
Column : 4 mm internal diameter × 500 mm (produced by Umetani Seiki)
Eluent : 30 v/v % acetonitrile in water
Flow rate : 1.0 ml/min
Pressure : 135 $kg/cm^2$
Chart speed : 1 cm/min

Under the above conditions, DL-mandelic acid was completely resolved. The separation is evidenced by the print-out of Figure 2, in which concentration along the ordinate is shown against eluting time along the abscissa. The separate peaks represent the eluted D- and L-isomers, with first peak in the chromatogram probably corresponding to the D isomer.

**Claims**

1. A material for resolution of optical isomers, the material comprising macromolecular silicon dioxide and molecules of a resolved optically active form of a cobalt(III) complex, said complex having six amino nitrogen atoms coordinated thereto, said cobalt(III) complex molecules being covalently bonded to said macromolecular silicon dioxide through a bridging chain.

2. A resolution material according to claim 1, wherein said macromolecular silicon dioxide is silica gel.

3. A resolution material according to claim 1 or 2, wherein each bridging chain extends to said macromolecular silicon dioxide from one of said six amino nitrogen atoms and includes one or more carbon atoms and a silicon atom, said silicon atom linking said carbon atoms to said macromolecular silicon dioxide.

4. A resolution material according to claim 1, 2 or 3, wherein said cobalt(III) complex comprises an optically active cation which can be represented by the formula (1) :

$$[Co(biN_2)\ (CoordN_4)]^{3+} \qquad (1)$$

wherein $biN_2$ represents a bidentate ligand, said bidentate ligand having two amino nitrogen atoms coordinated to the cobalt(III) atom and said bidentate ligand carrying on one of its nitrogen atoms said bridging carbon/silicon chain ; and $coordN_4$ represents four amino nitrogen atoms coordinated to the cobalt(III) atom and forming part of two bidentate ligands or one tetradentate ligand.

5. A material for optical resolution of a mixture of optical isomers of an optically active compound, said material comprising chromatographic silicon dioxide and a resolved optically active cobalt(III) complex, said complex being an optically active hexamine type complex with six amino nitrogen atoms coordinated to a cobalt(III) centre, there being a carbon chain attached to one of said six nitrogen atoms, said carbon chain connecting said cobalt(III) complex to said silicon dioxide through a silanol-type bond.

6. A compound of the general formula (2) :

$$(Si\text{-}O\text{-}Si\text{-})\text{-}A\text{-}[Co(biN_2)\ (CoordN_4)]^{3+} \cdot X$$

wherein :

(Si-O-Si-) represents a silicon dioxide macromolecule bonded through a silicon atom to the group A ;

A represents a divalent hydrocarbyl group ;

$Co(biN_2)$ $(coordN_4)$ is a resolved optically active cobalt(III) complex in which $biN_2$ represents a bidentate ligand, said bidentate ligand having two amino nitrogen atoms coordinated to the cobalt(III) atom and said bidentate ligand carrying on one of its nitrogen atoms said group A and $coordN_4$ represents four amino nitrogen atoms coordinated to the cobalt(III) atom and forming part of two bidentate ligands or one tetradentate ligand ; and

X represents one or more anions satisfying the cationic charge of 3 +.

7. A method for preparing a material for use in resolution of optical isomers, said material comprising macromolecular silicon dioxide and molecules of a resolved optically active form of a cobalt(III) complex, said complex having six amino nitrogen atoms coordinated thereto, said cobalt(III) complex molecules being covalently bonded to said macromolecular silicon dioxide through bridging chains ; which method comprises the steps of : resolving an optically active cobalt(III) complex having four coordinated amino nitrogen atoms and two coordinated labile atoms to give a resolved isomer, said four amino nitrogen atoms forming part of two bidentate ligands or one tetradentate ligand, and said two labile atoms forming part of two monodentate labile ligands or one bidentate labile ligand, each labile ligand being capable of ligand exchange with a bifunctional group having two coordinatable nitrogen atoms ; and reacting said resolved isomer of said optically active cobalt(III) complex in a solvent with a macromolecular silicon dioxide functionalized with an organic group having two nitrogen atoms capable of forming a bidentate ligand on the cobalt(III) complex.

8. A method for optical resolution of a mixture of optical isomers of an optically active compound, which method comprises contacting said mixture of optical isomers of an optically active compound with a resolution material comprising macromolecular silicon dioxide and molecules of a resolved optically active form of a cobalt(III) complex, said complex having six amino nitrogen atoms coordinated thereto, said cobalt(III) complex molecules being covalently bonded to said macromolecular silicon dioxide through bridging chains ; and sequentially separating said isomers from said material.

**Patentansprüche**

1. Material zur Spaltung von optischen Isomeren, welches makromolekulares Siliciumdioxid und Moleküle einer gespaltenen optisch aktiven Form eines Kobalt (III)-Komplexes umfaßt, wobei dieser Komplex sechs mit diesem koordinierte Stickstoffatome aufweist und die Moleküle des Kobalt (III)-Komplexes über eine brückenartige Kette kovalent an das makromolekulare Siliciumdioxid gebunden sind.

2. Material zur Spaltung nach Anspruch 1, in dem das makromolekulare Siliciumdioxid Silicagel ist.

3. Material zur Spaltung nach Anspruch 1 oder 2, in dem jede brückenartige Kette sich von einem der sechs Amino-Stickstoffatome zu dem makromolekularen Siliciumdioxid erstreckt und ein oder mehr Kohlenstoffatome und ein Siliciumatom umfaßt, wobei das Siliciumatom die Kohlenstoffatome mit dem makromolekularen Siliciumdioxid verbindet.

4. Material zur Spaltung nach Anspruch 1, 2 oder 3, in dem der Kobalt (III)-Komplex ein optisch aktives Kation aufweist, das durch die Formel (1) dargestellt werden kann :

$$[Co(biN_2)\ (coordN_4)]^{3+} \quad (1)$$

worin $biN_2$ einen zweizähligen Liganden darstellt, wobei der zweizählige Ligand zwei mit dem Kobalt (III)-Atom koordinierte Amino-Stickstoffatome aufweist und an einem seiner Stickstoffatome die brückenartige Kohlenstoff/Silicium-Kette trägt, und $coordN_4$ vier mit dem Kobalt (III)-Atom koordinierte Amino-Stickstoffatome bedeutet, welche einen Teil von zwei zweizähligen Liganden oder eines vierzähligen Liganden bilden.

5. Material zur optischen Spaltung eines Gemisches von optischen Isomeren einer optisch aktiven Verbindung, bestehend aus Siliciumdioxid für die Chromatographie und einem gespaltenen optisch aktiven Kobalt (III)-Komplex, wobei der Komplex ein optisch aktiver Hexammin-Komplex mit sechs Amino-Stickstoffatomen, die mit einem Kobalt (III)-Zentralatom koordiniert sind, ist, in dem eine Kohlenstoffkette an eines der sechs Stickstoffatome gebunden ist, welche den Kobalt (III)-Komplex über eine Silanol-Bindung mit dem Siliciumdioxid verknüpft.

6. Verbindung der allgemeinen Formel (2) :

$$(Si\text{-}O\text{-}Si\text{-})\text{-}A\text{-}[Co(biN_2)\ (coordN_4)]^{3+} \cdot X$$

in der

(Si-O-Si-) ein Siliciumdioxid-Makromolekül darstellt, welches über ein Siliciumatom an die Gruppe A gebunden ist ;

A eine zweiwertige Kohlenwasserstoffgruppe bedeutet ;

$Co(biN_2)$ $(coordN_4)$ ein gespaltener optisch aktiver Kobalt (III)-Komplex ist, wobei $biN_2$ einen zweizähligen Liganden bedeutet, welcher zwei mit dem Kobalt (III)-Atom koordinierte Amino-Stickstoffatome aufweist, wobei der zweizählige Ligand an einem seiner Stickstoffatome die Gruppe A trägt, und $coordN_4$ für vier Amino-Stickstoffatome steht, welche mit dem Kobalt (III)-Atom koordiniert sind und einen Teil von zwei zweizähligen Liganden oder eines vierzähligen Liganden bilden, und

X ein oder mehrere Anionen darstellt, welche die kationischen Ladung $3^+$ sättigen.

7. Verfahren zur Herstellung eines Materials zur Verwendung für die Spaltung von optischen Isomeren, welches makromolekulares Siliciumdioxid und Moleküle der gespaltenen optisch aktiven Form eines Kobalt (III)-Komplexes umfaßt, wobei dieser Komplex sechs koordinierte Amino-Stickstoffatome aufweist und die Moleküle des Kobalt (III)-Komplexes kovalent über brückenartige Ketten an das makromolekulare Siliciumdioxid gebunden sind, gekennzeichnet durch folgende Verfahrensschritte : Spalten eines optisch aktiven Kobalt (III)-Komplexes, der vier koordinierte Amino-Stickstoffatome und zwei koordinierte labile Atome aufweist, unter Bildung eines gespaltenen Isomeren, in welchem die vier Amino-Stickstoffatome einen Teil von zwei zweizähligen Liganden oder eines vierzähligen Liganden bilden und die zwei labilen Atome einen Teil von zwei einzähligen labilen Liganden oder eines zweizähligen labilen Liganden bilden und jeder labile Ligand zum Ligandenaustausch mit einer bifunktionellen Gruppe, die zwei koordinierbare Stickstoffatome aufweist, befähigt ist ; und Umsetzung des gespaltenen Isomeren des optisch aktiven Kobalt (III)-Komplexes in einem Lösungsmittel mit einem makromolekularen Siliciumdioxid, das mit einer organischen Gruppe mit zwei Stickstoffatomen, die zur Bildung eines zweizähligen Liganden an dem Kobalt (III)-Komplex befähigt ist, funktionalisiert ist.

8. Verfahren zur optischen Spaltung eines Gemisches von optischen Isomeren einer optisch aktiven Verbindung, bei dem das Gemisch aus optischen Isomeren einer optisch aktiven Verbindung mit einem Spaltungsmaterial in Berührung gebracht wird, welches aus makromolekularem Siliciumdioxid und Molekülen einer gespaltenen optisch aktiven Form eines Kobalt (III)-Komplexes besteht, wobei dieser Komplex sechs koordinierte Amino-Stickstoffatome aufweist und die Moleküle des Kobalt (III)-Komplexes kovalent über brückenartige Ketten an das makromolekulare Siliciumdioxid gebunden sind, und danach die Isomeren von dem Material abgetrennt werden.

**Revendications**

1. Matériau pour la résolution d'isomères optiques, comprenant un dioxyde de silicium macromoléculaire et des molécules d'une forme optiquement active, résolue, d'un complexe de cobalt(III), le complexe comportant 6 atomes d'azote aminé coordinés, les molécules de complexe de cobalt(III) étant reliées de manière covalente au dioxyde de silicium macromoléculaire par une chaîne de pontage.

2. Matériau de résolution selon la revendication 1, dans lequel le dioxyde de silicium macromoléculaire est un gel de silice.

3. Matériau de résolution selon l'une des revendications 1 et 2, dans lequel chaque chaîne de pontage s'étend vers le dioxyde de silicium macromoléculaire à partir de l'un des six atomes d'azote aminé et comprend un ou plusieurs atomes de carbone et un atome de silicium, l'atome de silicium reliant les atomes de carbone au dioxyde de silicium macromoléculaire.

4. Matériau de résolution selon l'une quelconque des revendications 1 à 3, dans lequel le complexe de cobalt (III) comprend un cation optiquement actif qui peut être représenté par la formule (1)

$$[Co(biN_2)\ (coordN_4)]^{3+} \qquad (1)$$

dans laquelle $BiN_2$ représente un ligand bidentate, le ligand bidentate ayant 2 atomes d'azote aminé coordinés à l'atome de cobalt(III) et le ligand bidentate portant sur l'un de ses atomes d'azote une chaîne de pontage carbone/silicium ; et $coordN_4$ représente 4 atomes d'azote aminé coordinés à l'atome de cobalt(III) et formant une partie de 2 ligands bidentate ou d'un ligand tétradentate.

5. Matériau pour la résolution optique d'un mélange d'isomères optiques d'un composé optiquement actif, le matériau comprenant une silice chromatographique et un complexe de cobalt(III) optiquement actif, résolu, le complexe étant un complexe du type hexamine, optiquement actif, avec six atomes d'azote aminé coordinés à un centre de cobalt(III), une chaîne carbonée étant fixée à l'un des 6 atomes d'azote, ladite chaîne carbonée reliant le complexe de cobalt(III) à la silice par une liaison de type silanol.

6. Composé de formule générale (2) :

$$(Si\text{-}O\text{-}Si)\text{-}A\text{-}[Co(biN_2)\ (coordN_4)]^{3+} \cdot X$$

dans laquelle :

(Si-O-Si-) représente une macromolécule de dioxyde de silicium reliée par un atome de silicium au groupe A ;

A représente un groupe hydrocarbyle divalent ;

Co($biN_2$) (coord$N_4$) est un complexe de cobalt(III) optiquement actif, résolu, dans lequel $biN_2$ représente un ligand bidentate, le ligand bidentate ayant 2 atomes d'azote aminé coordiné à l'atome de cobalt(III) et le ligand bidentate portant sur l'un de ses atomes d'azote le groupe A et coord$N_4$ représente 4 atomes d'azote aminé coordinés à l'atome de cobalt(III) et formant une partie de 2 ligands bidentates ou d'un ligand tétradentate ; et

X représente un ou plusieurs anions répondant à la charge cationique de 3 +.

7. Procédé pour la préparation d'un matériau utilisable dans la résolution d'isomères optiques, le matériau comprenant un dioxyde de silicium macromoléculaire et des molécules d'une forme optiquement active, résolue, d'un complexe de cobalt(III), le complexe comportant 6 atomes d'azote aminé coordinés, les molécules de complexe de cobalt(III) étant reliées de manière covalente au dioxyde de silicium macromoléculaire par l'intermédiaire de chaînes de pontage ; ledit procédé comportant les opérations qui consistent à : résoudre un complexe de cobalt(III) optiquement actif, ayant 4 atomes d'azote aminé coordinés et 2 atomes labiles coordinés pour obtenir un isomère résolu, les 4 atomes d'azote aminé formant une partie de 2 ligands bidentates ou d'un ligand tétradentate, et les deux atomes labiles formant une partie de 2 ligands labiles monodentates ou d'un ligand labile bidentate, chaque ligand labile étant capable d'échanger un ligand avec un groupe bifonctionnel ayant 2 atomes d'azote coordinables ; et à faire réagir l'isomère résolu du complexe de cobalt(III) optiquement actif, dans un solvant, avec un dioxyde de silicium macromoléculaire fonctionnalisé par un groupe organique ayant 2 atomes d'azote capables de former un ligand bidentate sur le complexe de cobalt(III).

8. Procédé pour la résolution optique d'un mélange d'isomères optiques d'un composé optiquement actif, lequel procédé consiste à mettre en contact le mélange d'isomères optiques d'un composé optiquement actif avec un matériau de résolution comprenant du dioxyde de silicium macromoléculaire et des molécules d'une forme optiquement active, résolue, d'un complexe de cobalt(III), le complexe ayant 6 atomes d'azote aminé coordinés, les molécules de complexe de cobalt(III) étant reliées de manière covalente au dioxyde de silicium macromoléculaire par des chaînes de pontage ; et à séparer ensuite les isomères du matériau.

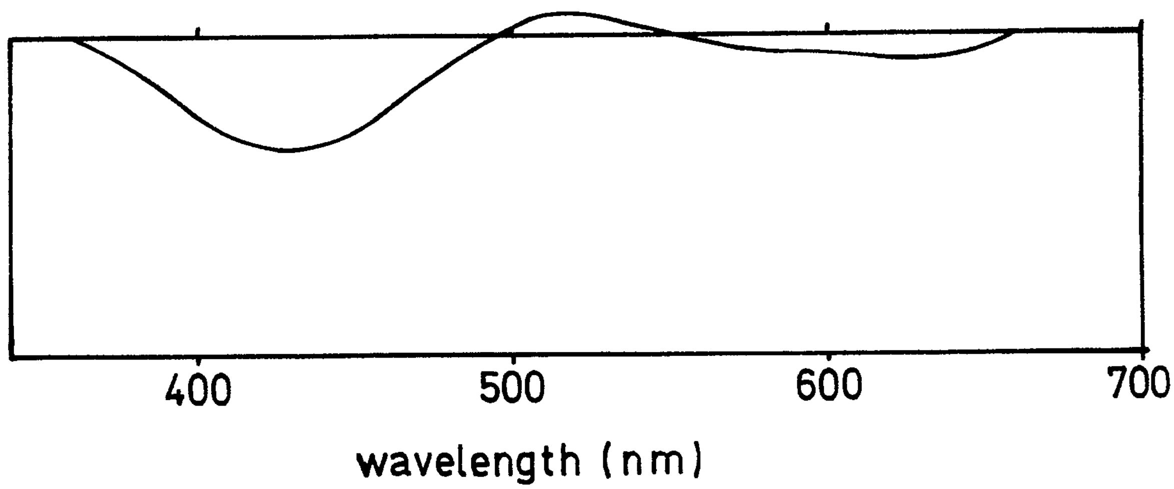


FIG.1.

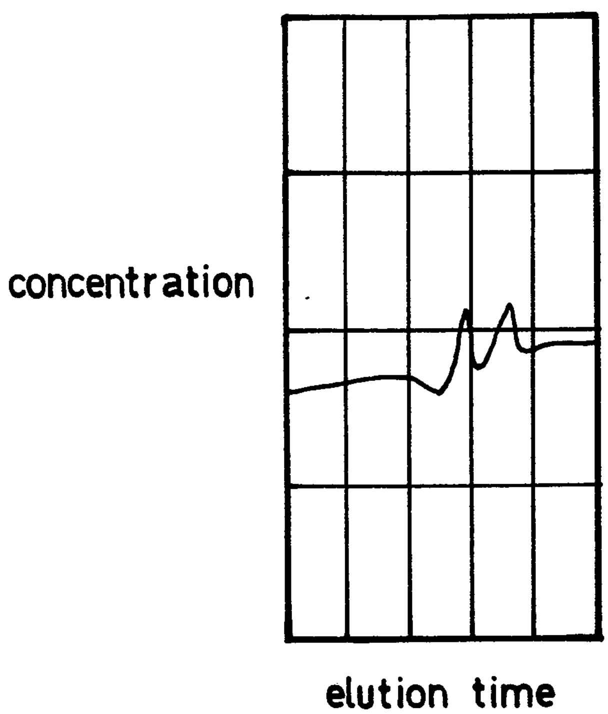


FIG.2.